(19) Europäisches Patentamt
European Patent Office
Office européen des brevets

(11) Publication number: **0 080 819**
**B1**

(12) **EUROPEAN PATENT SPECIFICATION**

(45) Date of publication of patent specification: **16.01.85**

(51) Int. Cl.$^4$: **C 07 H 17/08,** A 61 K 31/70

(21) Application number: **82306036.3**

(22) Date of filing: **12.11.82**

(54) **11-0-Alkylerythromycin A derivatives.**

(30) Priority: **30.11.81 JP 192294/81**

(43) Date of publication of application:
**08.06.83 Bulletin 83/23**

(45) Publication of the grant of the patent:
**16.01.85 Bulletin 85/03**

(84) Designated Contracting States:
**CH DE FR GB IT LI NL**

(56) References cited:
**US-A-3 884 904**
**US-A-3 884 904**

(73) Proprietor: **TAISHO PHARMACEUTICAL CO. LTD**
**24-1 Takata 3-chome Toshima-ku**
**Tokyo 171 (JP)**

(72) Inventor: **Morimoto, Shigeo**
**121-9, Homura Yoshikawa-cho**
**Kitakatsushika-gun Saitama 342 (JP)**
Inventor: **Watanabe, Yoshiaki**
**Ogawadanchi 1-101, 2786 Ogawahigashi-cho**
**Kodaira Tokyo 187 (JP)**
Inventor: **Takahashi, Yoko**
**Gurin-Haitsu 101**
**1216-10 Kawarabuki Ageo Saitama 362 (JP)**
Inventor: **Omura, Sadafumi**
**523-7 Haraichi**
**Ageo Saitama 362 (JP)**

(74) Representative: **Evans, David Charles et al**
**F.J. CLEVELAND & COMPANY 40-43, Chancery Lane**
**London, WC2A 1JQ (GB)**

**EP 0 080 819 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to novel antibacterial agents. More specifically, it is concerned with novel 11-O-alkylerythromycin A derivatives having *in vivo* antibacterial activity against Gram-positive bacteria and Mycoplasma species.

The present invention is based on the discovery that new erythromycin A derivatives having 11-O-alkyl substituents exhibit higher *in vivo* antibacterial activity than other closely analogous compounds such as erythromycin A against Gram-positive bacteria.

The compounds of the present invention are an erythromycin A derivative of the general formula

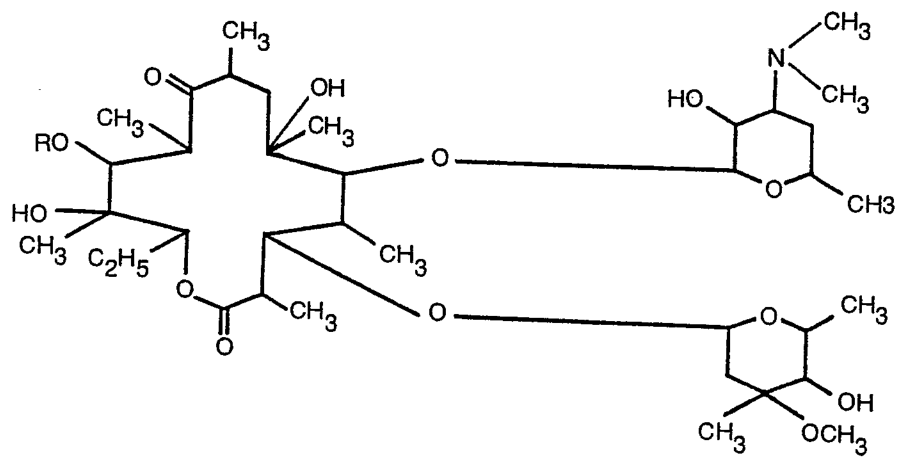

I

wherein R is alkyl having 1—3 carbon atoms, and pharmaceutically acceptable acid addition salts thereof.

Most preferred compound of the present invention is that of formula I wherein R is methyl group.

The pharmaceutically acceptable acid addition salts of the compounds of formula I include salts with inorganic acids such as hydrochloric acid, hydrobromic acid, hydroiodic acid, nitric acid, sulfuric acid, sulfurous acid and phosphoric acid or organic acids such as formic acid, acetic acid, propionic acid, butyric acid, lactic acid, citric acid, malic acid, glycolic acid, tartaric acid, succinic acid, maleic acid, fumaric acid, gluconic acid, stearic acid, mandelic acid, benzoic acid, methanesulfonic acid, aminoethanesulfonic acid, p-toluenesulfonic acid, glutamic acid, and aspartic acid.

The compounds of formula I may be prepared, for example, by the following processes: Namely, a compound of the formula

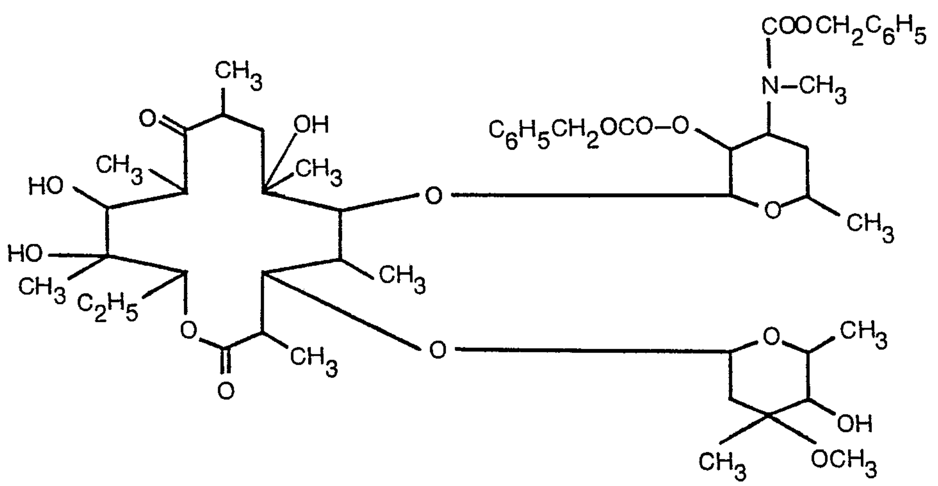

II

is reacted with an alkylating agent such as methyl iodide, ethyl iodide, propyl iodide, dimethyl sulfate or methyl p-toluenesulfonate, in the presence of a suitable base in a solvent in order to effect alkylation of the compound of formula II to give a compound of the formula

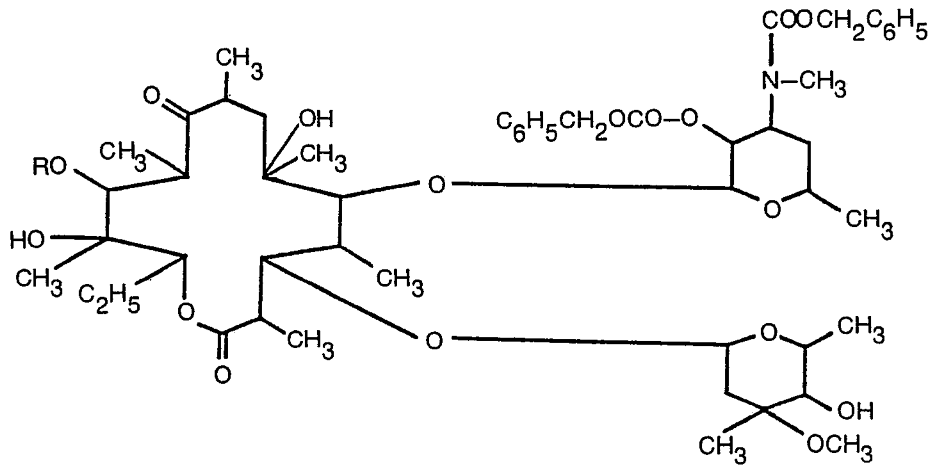

III

wherein R is defined above.

Examples of the base used in alkylation are alkali metal hydrides, e.g., lithium hydride, sodium hydride or potassium hydride; alkali metal amides, e.g., lithium amide, sodium amide or potassium amide; butyllithium and lithium diisopropylamide.

The solvents used include inert solvents in which the compound of formula II is dissolved, for example, polar aprotic solvents such as N,N-dimethylformamide, N,N-dimethylacetamide, dimethylsulfoxide and hexamethylphosphoric triamide, preferably N,N-dimethylformamide.

In alkylation, 5—10 moles of the alkylating agent, and 1—2 moles of the base are employed per mole of the compound of formula II. The reaction proceeds at the temperature ranging from −78°C to room temperature, preferably from −10°C to 5°C.

The compound of formula III thus obtained may be worked up in a conventional manner, if desired, further purified by recrystallization or column chromatography.

The compound of formula III is subjected to catalytic reduction according to the method reported by E. H. Flynn et al. in Journal of the American Chemical Society, *77*, page 3104(1955) in order to eliminate the benzyloxycarbonyl groups, and then subjected to further catalytic reduction in the presence of excess formaldehyde to effect N-methylation, thus giving the compound of formula I.

The intermediate thus obtained by catalytic reduction for the above elimination may be used without isolation, or after isolation in a conventional manner for N-methylation.

Alternatively, when an alkylating agent is used which forms only with difficulty a quaternary ammonium salt with the 3′-dimethylamino group, the compound of formula I may be directly prepared by alkylation of erythromycin A with the above base and solvent.

Purification of the compound of formula I may be carried out by recrystallisation or column chromatography.

The pharmaceutically acceptable acid addition salt of the compound of formula I may be obtained by treating the compound of formula I with one mole equivalent of the corresponding acid described above in an inert solvent such as water, acetone, methanol and ethanol. The salts thus obtained are collected by filtration if they are insoluble in the inert solvent, by precipitation by addition of a nonsolvent for the salt, or by evaporation of the solvent.

The starting material of formula II can be prepared according to the above-described method of E. H. Flynn et al.

The compounds of the present invention have excellent *in vivo* antibacterial activity against Gram-positive bacteria and Mycoplasma species, therefore, they can be used as the antibacterial agents in mammals. For these purposes, a compound of formula I may be administered orally or parenterally in a conventional dosage form such as tablet, capsule, powder, troche, dry mixes, ointment, suspension or solution prepared according to conventional pharmaceutical practices.

The compounds of formula I can be administered at a dosage of from about 1 mg/kg to about 100 mg/kg of body weight per day. The preferred dosage range is from about 2 mg/kg to about 25 mg/kg of body weight per day.

The compounds of the present invention have excellent low toxicity. The $LD_{50}$ value in mice is in excess of 5000 mg/kg of body weight.

Following is a description by way of example only of methods of carrying the invention into effect.

## Example 1

(1) In 80 ml of N,N-dimethylformamide were dissolved 19.8 g of 2′-O-benzyloxycarbonyl-N-benzyloxycarbonyl-des-N-methylerythromycin A and 8.4 ml of methyl iodide. The solution was stirred under cooling at 0—2°C in a nitrogen stream and 1.21 g of 50% sodium hydride dispersion were added thereto in small portions. Stirring was continued for a further 30 minutes.

After completion of the reaction, 25 ml of triethylamine were poured into the reaction mixture with stirring under ice-cooling, and 300 ml of ethyl acetate and 150 ml of a saturated aqueous sodium chloride solution were added. The ethyl acetate layer was collected, washed with a saturated aqueous sodium chloride solution, and dried over anhydrous magnesium sulfate.

After evaporation of the solvent, the residue was then applied to a column chromatography (silica gel 60 for column chromatography produced by E. Merck Darmstadt, 70—230 mesh, $\phi$ 5.4 x 50 cm; and a mixture of ethyl acetate and n-hexane(1:1) as a developing solvent), and 15 ml each of fractions were collected. Thin layer chromatography (silica gel 60 $F_{254}$ produced by E. Merck Darmstadt, and a mixture of ethyl acetate and n-hexane(1:1) as a developing solvent) was applied to the detection of the product for each fraction, the fractions having a spot at Rf value of 0.09 were collected (cf., Rf value of the starting material 0.07), and the solvent was evaporated in vacuo to give 14.2 g of a colorless foam. Recrystallization from ether gave 11.7 g of the compound of formula III wherein R is methyl.

m.p. 150.5—153.5°C (with decomposition)

Mass (m/e): 1001 ($M^+$)

IR $\nu_{max}^{KBr}$ cm$^{-1}$: 3520, 3500, 3420, 1755, 1732, 1703, 1685

$^1$H-NMR($CDCl_3$): $\delta = 3.47$(3H)

(2) In 10 ml of dry N,N-dimethylformamide were dissolved 2 g of 2'-O-benzyloxycarbonyl-N-benzyloxycarbonyl-des-N-methylerythromycin A and 1 ml of dimethyl sulfate. The solution was stirred under cooling at 0—2°C in a nitrogen stream, and 115 mg of 50% sodium hydride dispersion was added thereto in small portions. Stirring was continued for a additional 30 minutes.

After completion of the reaction, the reaction mixture was worked up by the similar procedure to that of item(1) to give 1.4 g of the same compound as obtained in item(1).

(3) In 20 ml of a mixture of dry dimethylsulfoxide and tetrahydrofuran(1:1) were dissolved 1.7 g of 2'-O-bezyloxycarbonyl-N-benzyloxycarbonyl-des-N-methylerythromycin A and 1 ml of methyl iodide. Methylation was carried out by the same procedure as that of item(1), except for the use of 192 mg of 50% sodium hydride dispersion in place of 1.21 g thereof to give 0.9 g of the same compounds as obtained in item(1).

(4) In a mixture of 1.2 ml of acetic acid, 60 ml of water and 300 ml of ethanol were dissolved 6 g of the compound of formula III obtained in item(1) and 2 g of sodium acetate. 0.6 g of palladium black was added, and the mixture was stirred for 5 hours at ambient temperature under atmospheric pressure in a gentle hydrogen stream to effect catalytic reduction. Into the resulting mixture was poured 20 ml of 37% aqueous formaldehyde solution, and catalytic reduction was continued for a further 7 hours.

After completion of the reaction, the catalyst was filtered off and the filtrate was concentrated in vacuo to approximately a quarter volume, to which were added 100 ml of water and an aqueous sodium carbonate solution to adjust to about pH 10. The mixture was extracted thoroughly with dichloromethane, the extract was washed with water and dried, and the solvent was distilled off.

The residue thus obtained was recrystallized from a mixture of acetone and petroleum ether, giving 3.73 g of 11-O-methylerythromycin A.

m.p. 182—185°C (with decomposition)

Elemental analysis (for $C_{38}H_{69}NO_{13}$)

| | | | |
|---|---|---|---|
| Calcd. (%): | C, 61.02; | H, 9.30; | N, 1.87 |
| Found. (%): | C, 61.09; | H, 9.46; | N, 1.82 |

FD-Mass (m/e): 747 ($M^+$)

IR $\nu_{max}^{KBr}$ cm$^{-1}$: 3500, 3440, 1738, 1732, 1705

$^1$H-NMR($CDCl_3$): $\delta = 2.30$, 2.32(6H), 3.30, 3.35, 3.37, 3.48(6H)

---

$^1$H-NMR data indicates that this product is a mixture of the tautomers, ie., hydroxyketone and hemi-acetal, when being dissolved in $CDCl_3$.

Example 2

In 40 ml of dry N,N-dimethylformamide were dissolved 7.3 g of erythromycin A and 4 ml of ethyl iodide. The solution was stirred under cooling at 0—5°C in a nitrogen stream, and 594 mg of 50% sodium hydride dispersion was added thereto in small portions with stirring. Stirring was continued for a further 30 minutes.

After completion of the reaction, 10 ml of triethylamine were poured into the reaction mixture under ice-cooling, and 200 ml of ethyl acetate and 100 ml of a saturated aqueous sodium chloride solution were added. The ethyl acetate layer was collected, washed with a saturated aqueous sodium chloride solution, and dried over anhydrous magnesium sulfate. Evaporation of the solvent left the residue, which was then subjected to silica gel column chromatography (silica gel 60 silanized for column chromatography produced by E. Merck Darmstadt, 70—230 mesh; and a mixture of chloroform and methanol(9:1) as a developing solvent) to give a colorless foam. Recrystallization from a mixture of acetone and petroleum ether gave 3.1 g of 11-O-ethylerythromycin A.

m.p. 123.5—126.5°C (with decomposition)

Elemental analysis (for $C_{39}H_{71}NO_{13}$)

| | | | |
|---|---|---|---|
| Calcd. (%): | C, 61.47; | H, 9.39; | N, 1.84 |
| Found. (%): | C, 61.13; | H, 9.29; | N, 1.83 |

Mass (m/e): 761 ($M^+$)
IR $\nu_{max}^{KBr}$ $cm^{-1}$: 3460, 1735
$^1H$-NMR($CDCl_3$): $\delta = 2.38$, 2.33(6H), 3.38, 3.33(3H)
$^1H$-NMR data indicates that this product is a mixture of the tautomers, ie., hydroxyketone and hemi-acetal, when being dissolved in $CDCl_3$.

Example 3

Following the similar treatment to that of Example 2 using 11 g of erythromycin A and 16 g of n-propyl iodide, 6.6 g of 11-O-n-propylerythromycin A as crystals.
m.p. 121.5—125.5°C
Elemental analysis (for $C_{40}H_{73}NO_{13}$)

| | | | |
|---|---|---|---|
| Calcd. (%): | C, 61.91; | H, 9.48; | N, 1.81 |
| Found. (%): | C, 61.63; | H, 9.39; | N, 1.76 |

Mass (m/e): 775 ($M^+$)
IR $\nu_{max}^{KBr}$ $cm^{-1}$: 3440, 1730
$^1H$-NMR($CDCl_3$): $\delta = 2.32$, 2.34(6H), 3.32, 3.37(3H)
$^1H$-NMR data indicates that this product is a mixture of the tautomers, ie., hydroxyketone and hemi-acetal, when being dissolved in $CDCl_3$.

The following experiment illustrates *in vivo* antibacterial activity of the compound of the present invention.

Experiment

Male ddY mice weighing 20—23 g in groups 20 each were inoculated with *Staphylococcus aureus* Smith No. 4 ($10^7$ cells per mouse, i.p.). Erythromycin A was used as a control, the compound of formula I wherein R is methyl group("compound 1") was administered orally at one hour after inoculation, and the number of living mice for seven days after administration was calculated to determine the *in vivo* antibacterial activity. The results are shown in the following table.

| *In vivo* Antibacterial Activity [The Number of Surviving Mice] | | |
|---|---|---|
| Test Compound / dose (mg/mouse) | Compound 1 | Erythromycin A |
| 4 | 20 | 20 |
| 2 | 20 | 18 |
| 1 | 12 | 8 |
| 0.5 | 6 | 4 |

## Claims

1. An erythromycin A derivative of the formula

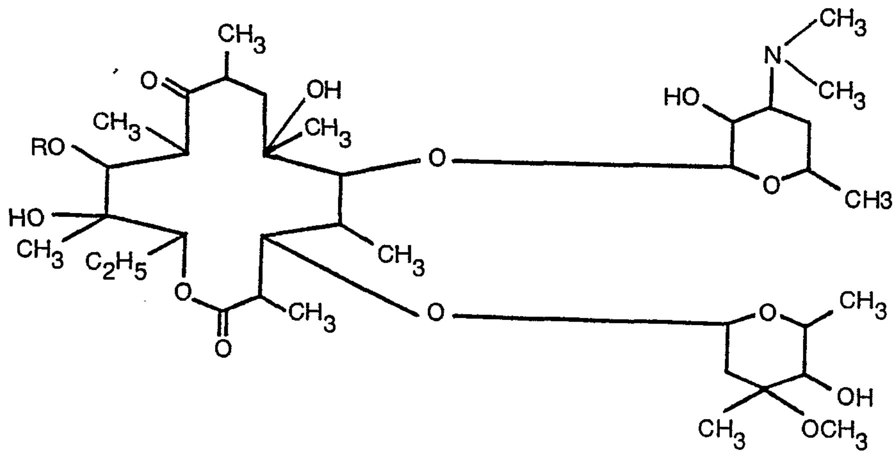

wherein R is alkyl having 1 to 3 carbon atoms, and the pharmaceutically acceptable acid addition salts thereof.

2. A compound according to claim 1 wherein R is methyl group.

3. A compound according to claim 1 wherein R is ethyl group.

4. A compound according to claim 1 wherein R is propyl group.

5. A compound as claimed in any preceding claim wherein the compound is in the form of an addition salt selected from one or more of hydrochloric acid, hydrobromic acid, hydroiodic acid, nitric acid, sulfuric acid, sulfurous acid, phosphoric acid, formic acid, acetic acid, propionic acid, butyric acid, lactic acid, citric acid, malic acid, glycolic acid, tartaric acid, succinic acid, maleic acid, fumaric acid, gluconic acid, stearic acid, mandelic acid, benzoic acid, methanesulfonic acid, aminoethanesulfonic acid, p-toluenesulfonic acid, glutamic acid and aspartic acid.

6. A pharmaceutical composition comprising a compound as claimed in any preceding claim together with a pharmaceutically inert diluent therefor.

7. A composition as claimed in claim 6 wherein the dosage is 1 mg to 100 mg/kg body weight per day.

8. A method of preparing the derivative of claim 1 wherein a compound of formula II

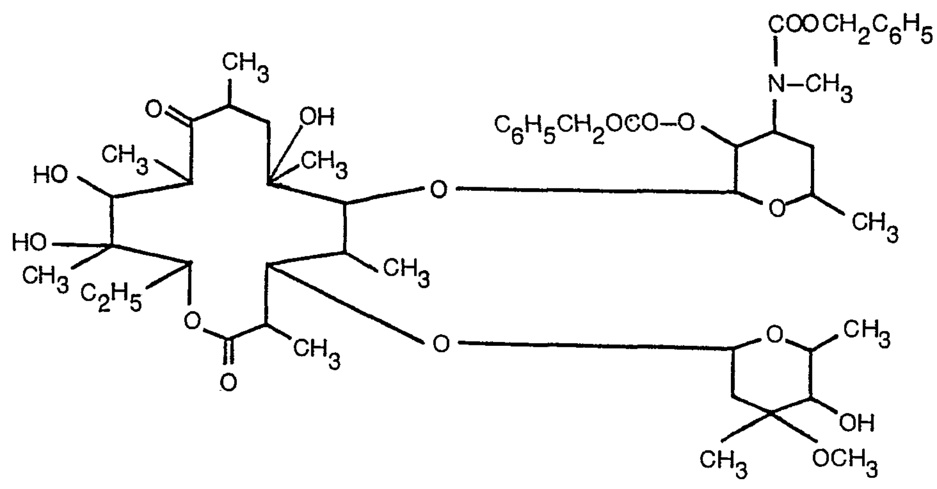

II

is reacted with an alkylating agent in the presence of a suitable base in a solvent to obtain an intermediate compound of the formula III

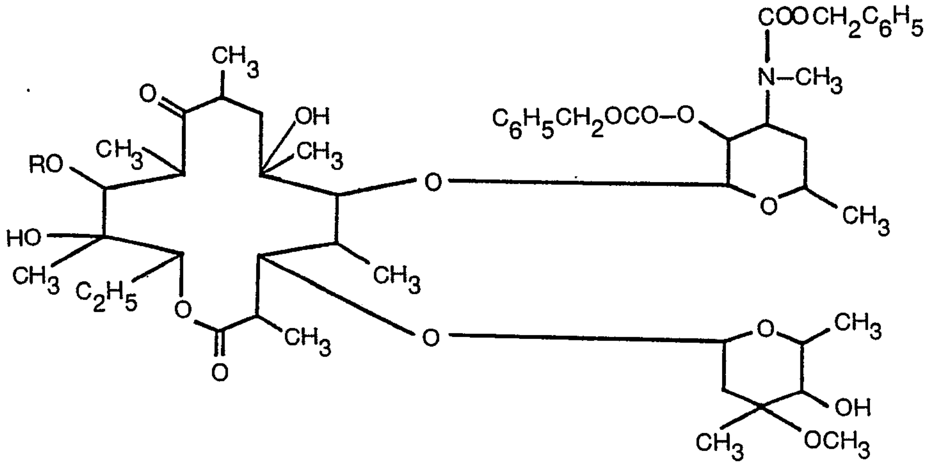

III

wherein R is as defined in claim 1 and thereafter subjecting said intermediate to catalytic reduction to eliminate the benzyloxycarbonyl groups and then effecting further catalytic reduction in the presence of excess formaldehyde to effect N-methylation to produce a compound in accordance with formula I of claim 1.

9. A method as claimed in claim 9 wherein in alkylation 5 to 10 moles of alkylating agent and 1 to 2 moles of base per mole of compound of formula II are employed.

10. A method as claimed in claim 8 or claim 9 wherein the alkylation reaction is carried out at temperatures from —78°C to ambient temperature.

**Patentansprüche**

1. Erythromycin-A-Derivat der Formel

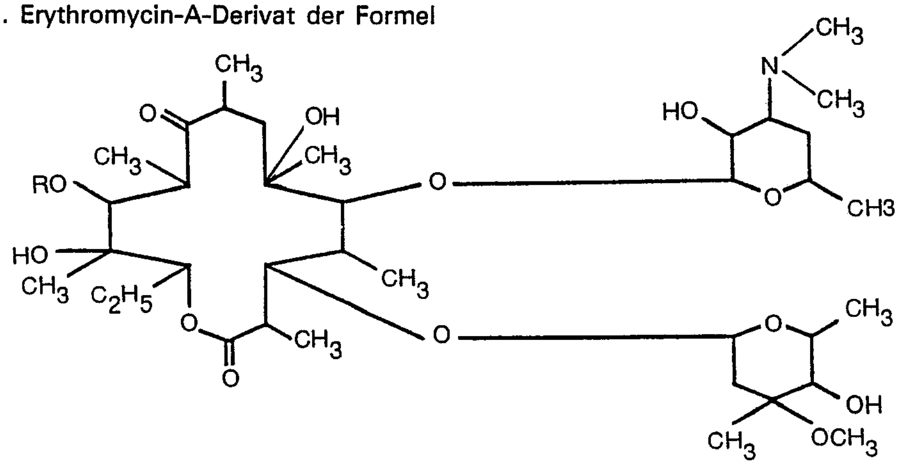

in der R Alkyl mit 1 bis 3 Kohlenstoffatomen ist, sowie dessen pharmazeutisch annehmbare Säureadditions-Salze.

2. Verbindung nach Anspruch 1, in der R eine Methylgruppe ist.

3. Verbindung nach Anspruch 1, in der R eine Ethylgruppe ist.

4. Verbindung nach Anspruch 1, in der R eine Propylgruppe ist.

5. Verbindung nach einem beliebigen vorausgehenden Anspruch, worin die Verbindung in Form eines Additionssalzes vorliegt, das ausgewählt ist aus einer oder mehreren der Säuren Chlorwasserstoffsäure, Bromwasserstoffsäure, Jodwasserstoffsäure, Salpetersäure, Schwefelsäure, schweflige Säure, Phosphorsäure, Ameisensäure, Essigsäure, Propionsäure, Buttersäure, Milchsäure, Zitronensäure, Apfelsäure, Glykolsäure, Weinsäure, Bernsteinsäure, Maleinsäure, Fumarsäure, Glukonsäure, Stearinsäure, Mandelsäure, Benzoesäure, Methansulfonsäure, Aminoethansulfonsäure, p-Toluolsulfonsäure, Glutaminsäure und Asparaginsäure.

6. Pharmazeutische Zusammensetzung mit einer Verbindung, wie sie in einem beliebigen vorausgehenden Anspruch beansprucht wird, zusammen mit einem pharmazeutisch inerten Verdünnungsmittel für diese.

7. Zusammensetzung nach Anspruch 6, bei der die Dosierung 1 mg bis 100 mg/kg Körpergewicht pro Tag beträgt.

8. Verfahren zur Herstellung des Derivats gemäß Anspruch 1, bei dem eine Verbindung der Formel II

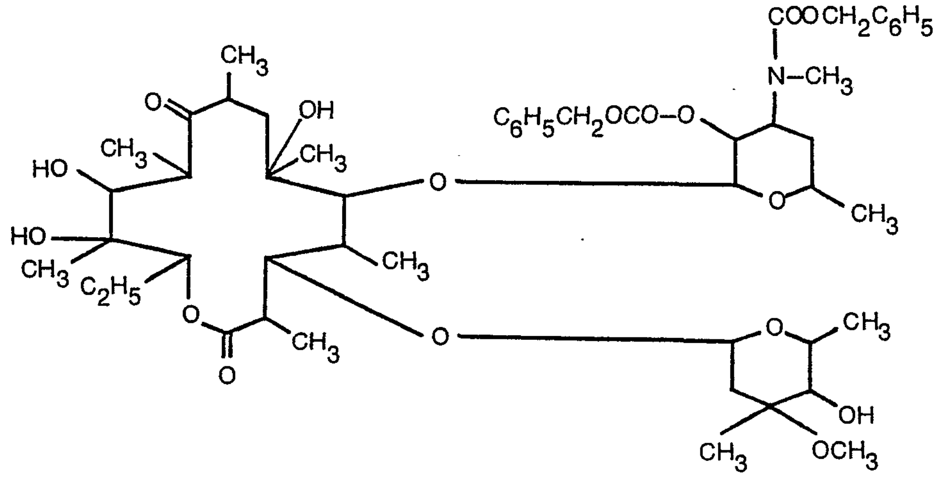

II

mit einem Alkylierungsmittel in Gegenwart einer geeigneten Base in einem Lösungsmittel umgesetzt wird, um eine Zwischenstufen-Verbindung der Formel III zu erhalten

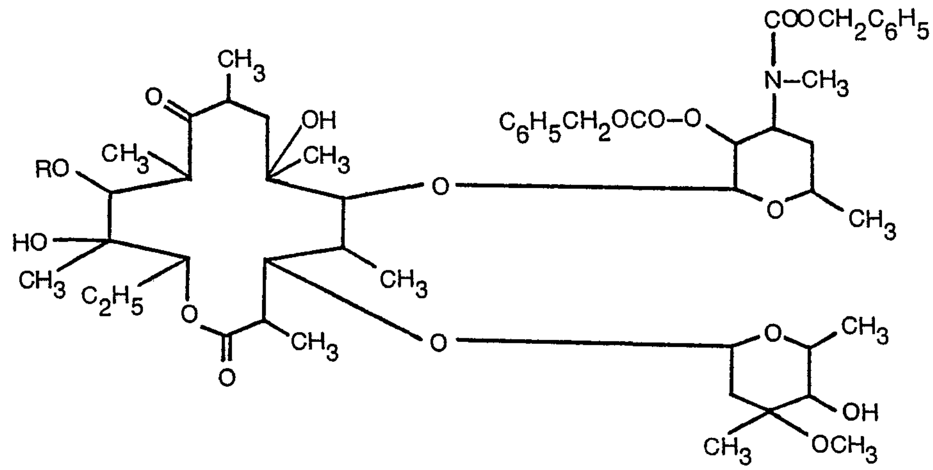

III

in der R wie in Anspruch 1 definiert ist, und danach die genannte Zwischenstufe einer katalytischen Reduktion unterzogen wird, um die Benzyloxycarbonyl-Gruppen zu entfernen und bei dem anschließend eine weitere katalytische Reduktion in Gegenwart eines Überschusses Formaldehyd bewirkt wird, um eine N-Methylierung unter Herstellung einer Verbindung gemäß Formel I aus Anspruch 1 zu bewirken.

9. Verfahren nach Anspruch 8, bei dem bei der Alkylierung 5 bis 10 Mol des Alkylierungsmittels und 1 bis 2 Mol Base pro Mol der Verbindung der Formel II verwendet werden.

10. Verfahren nach Anspruch 8 oder Anspruch 9, bei dem die Alkylierungsreaktion bei Temperaturen von $-78°C$ bis Umgebungstemperatur durchgeführt wird.

## Revendications

1. Dérivé d'érythromycine A de formule

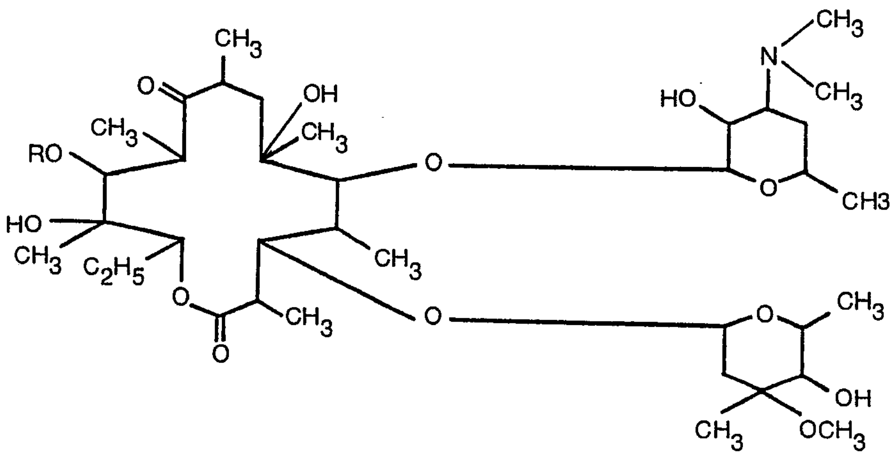

où R est un alcoyle en $C_1$ à $C_3$, et ses sels d'addition d'acides pharmaceutiquement acceptables.

2. Composé selon la revendication 1 où R est un groupe méthyle.

3. Composé selon la revendication 1 où R est un groupe éthyle.

4. Composé selon la revendication 1 où R est un groupe propyle.

5. Composé selon l'une quelconque des revendications précédentes où le composé est sous la forme d'un sel d'addition d'acide choisi parmi un ou plusieurs des acides chlorhydrique, bromhydrique, iodhydrique, nitrique, sulfurique, sulfureux, phosphorique, formique, acétique, propionique, butyrique, lactique, citrique, malique, glycolique, tartrique, succinique, maléique, fumarique, gluconique, stéarique, mandélique, benzoïque, méthanesulfonique, aminoéthanesulfonique, p-toluènesulfonique, glutamique et aspartique.

6. Composition pharmaceutique comprenant un composé selon l'une quelconque des revendications précédentes avec un diluant pharmaceutiquement inerte approprié.

7. Composition selon la revendication 6 où la posologie est de 1 mg à 100 mg/kg de poids corporel par jour.

8. Procédé de préparation du dérivé de la revendication 1 où l'on fait réagir un composé de formule II

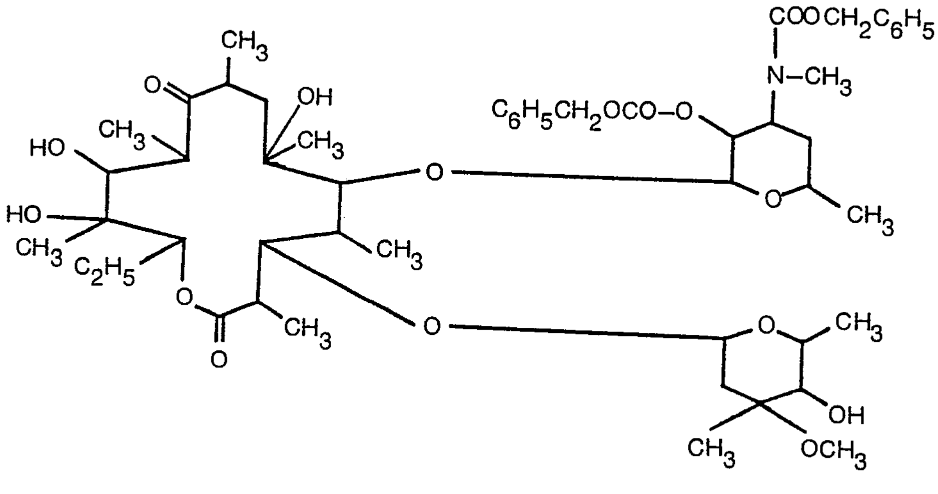

II

avec un agent alcoylant en présence d'une base appropriée dans un solvant pour obtenir un composé intermédiaire de formule III

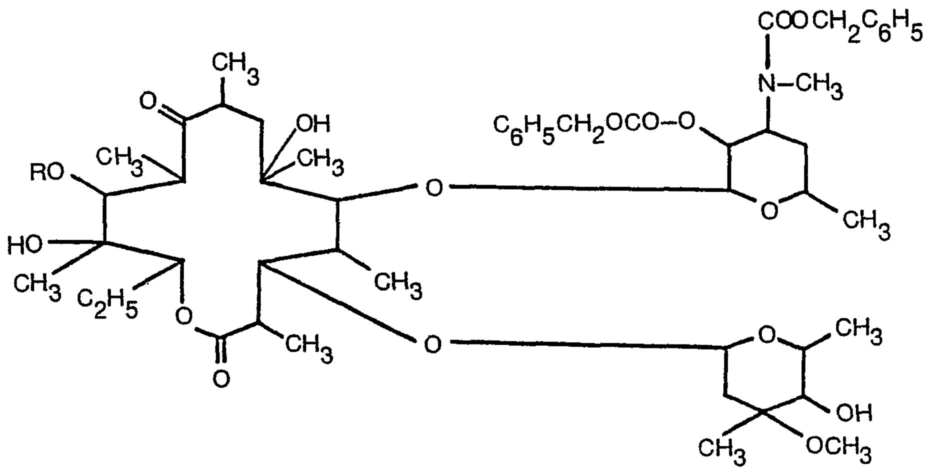

III

où R est tel que défini dans la revendication 1 puis on soumet ledit intermédiaire à une réduction catalytique pour éliminer les groupes benzyloxycarbonyle, puis on effectue une autre réduction catalytique en présence d'un excès de formaldéhyde pour effectuer une N-méthylation afin de produire un composé selon la formule I de la revendication 1.

9. Procédé selon la revendication 9 où dans l'alcoylation ou emploie de 5 à 10 moles d'agent alcoylant et de 1 à 2 moles de base par mole de composé de formule II.

10. Procédé selon les revendications 8 et 9 où la réaction d'alcoylation est conduite à des températures allant de —78°C à la température ambiante.